**Europäisches Patentamt**

**European Patent Office**

**Office européen des brevets**

(11) Publication number: **0 085 577**

**B1**

(12) **EUROPEAN PATENT SPECIFICATION**

(45) Date of publication of patent specification: **16.06.87**

(21) Application number: **83300528.3**

(22) Date of filing: **02.02.83**

(51) Int. Cl.⁴: **A 61 B 5/02, A 61 B 5/04**

(54) A heart-beat rate indicator.

(30) Priority: **03.02.82 JP 15660/82**

(43) Date of publication of application:
**10.08.83 Bulletin 83/32**

(45) Publication of the grant of the patent:
**16.06.87 Bulletin 87/25**

(84) Designated Contracting States:
**DE FR GB**

(56) References cited:
**AT-A- 358 127**
**DE-A-2 809 935**
**DE-A-2 847 087**
**DE-A-3 018 604**
**FR-A-1 359 691**
**FR-A-2 195 341**
**GB-A-2 109 559**

(73) Proprietor: **KABUSHIKI KAISHA DAINI SEIKOSHA**
**31-1, 6-chome Kameido**
**Koto-ku Tokyo (JP)**

(72) Inventor: **Tabata, Junichi**
**c/o Kabushiki Kaisha Daini Seikosha**
**6-31-1, Kameido Koto-ku Tokyo (JP)**

(74) Representative: **Caro, William Egerton et al**
**J. MILLER & CO. Lincoln House 296-302 High**
**Holborn**
**London WC1V 7JH (GB)**

## Description

This invention relates to heart-beat rate indicators or pulsimeters.

Conventionally, heart-beat rate is measured by electro-cardiography where a small electric signal produced by the heart prior to contraction is detected. It has been proposed to measure heart-beat rate by detecting the electrocardiac potential signal induced between two metal electrodes in contact with different parts of the human body. Theoretically, it should be possible to provide a small size and long life heart-beat rate indicator· because the electrocardiac potential signal can be detected with low power dissipation e.g. 100µw.

DE—A—2847087 discloses a heart-beat rate indicator comprising a detection electrode for receiving an electrocardiac potential signal and circuitry for processing the electrocardiac potential signal to determine heart-beat rate therefrom. However, this construction is such that the input of the circuitry may be destroyed or damaged by electrostatic charge built up on, for example, clothing and transferred to the heart-beat rate indicator upon touching one of the electrodes.

According to the present invention there is provided a heart-beat rate indicator comprising: a detection electrode for receiving an electrocardiac potential signal; and circuit means for receiving the electrocardiac potential signal for determining therefrom a heart-beat; characterised by a switching element one side of which is connected to the detection electrode and an input of the circuit means and the other side of which is connectable to ground potential, switching means for controlling the conductive state of the switching element, the switching means including a mechanical switch operated by pressure applied to the detection electrode and arranged to ensure electrostatic charge transferred to the detection electrode is eliminated before the mechanical switch is actuated.

The switching element may be a P-channel MOSFET.

The switching means may include memory means connected to control the conductive state of the switching element.

In one embodiment the memory means is connected to control the conductive state of the circuit means. Preferably the memory means is a flip-flop circuit.

The invention is illustrated, merely by way of example, in the accompanying drawings, in which:—

Figure 1 is a waveform of the human heart-beat;

Figure 2 is a schematic diagram of a conventional heart-beat rate indicator;

Figure 3 is a block diagram of one embodiment of a heart-beat rate indicator according to the present invention;

Figure 4 is a block diagram of another embodiment of a heart-beat rate indicator according to the present invention;

Figure 5 is a plan view of a display device of a heart-beat rate indicator according to the present invention; and

Figure 6 is a sectional view of a detection electrode and a switch of the heart-beat rate indicators of Figures 3 and 4.

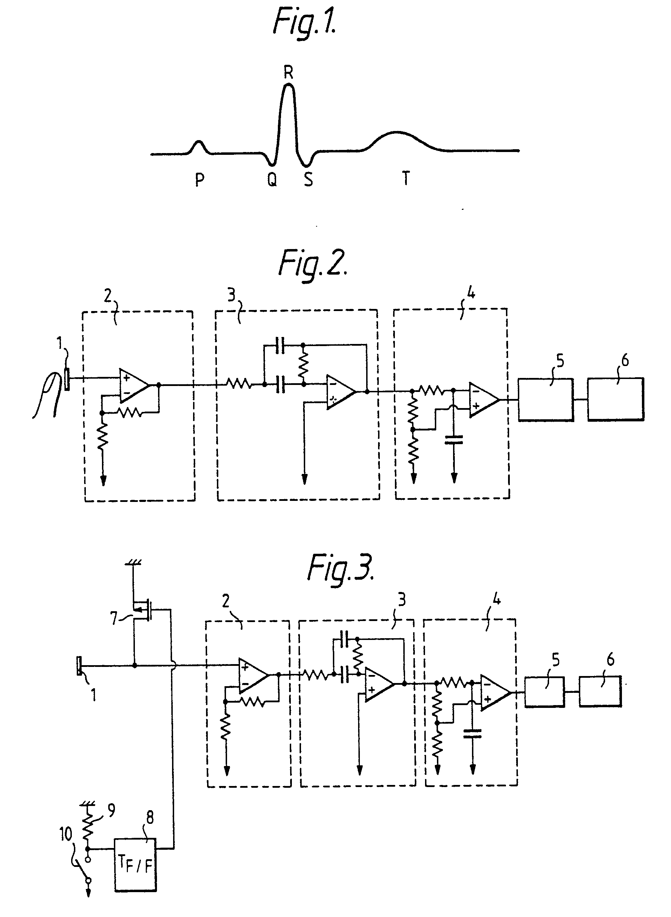

Figure 1 shows a waveform of a human heart-beat. Generally, an electrocardiac potential signal induced between the arms of a person is composed of a P-wave, a Q—R—S-wave and a T-wave, these appearing periodically. The amplitude of the Q—R—S-wave is the largest of the three and ranges from about 0.2 mV to 1.0 mV. Normally heart-beat rate indicators detect the Q—R—S-wave.

On top of the electrocardiac signal is superimposed noise at commercial frequencies induced on the surface of the human body from outside. For heart-beat rate measurement it is necessary to eliminate large amplitude noise in order to detect the electrocardiac potential signal which is of small amplitude.

A conventional heart-beat rate indicator is illustrated in Figure 2. A detection electrode 1 is composed of, for example, stainless steel or silver chloride. When a person measures heart-beat rate, a portion of the body surface, for example, the skin of one arm (left arm) acts as ground potential and the skin of the other arm (right arm) for example the tip of a finger, contacts the detection electrode 1. Thus the heart-beat rate indicator receives an electrocardiac potential signal. The ground potential may be applied to a casing of the heart-beat rate indicator when it is worn on the wrist.

The detection electrode 1 is connected to an amplifier circuit 2. The amplifier circuit 2 is an operational amplifier with a plurality of resistors so that it can have a desired amplification factor.

The electrocardiac potential signal and noise is amplified by the amplifier circuit 2 to the desired level and fed to a band-pass filter 3. The band-pass filter 3 consists of an operational amplifier, a plurality of resistors and a plurality of capacitors and has both a predetermined centre frequency and a predetermined Q-value. Noise at commercial frequencies is eliminated by the band-pass filter 3 so that only the electrocardiac potential signal appears at the output of the band-pass filter. The output from the band-pass filter 3 is fed to a voltage comparator 4. The voltage comparator 4 detects only the Q—R—S wave of the electrocardiac potential signal and produces a pulse signal at its output. The pulse signal from the voltage comparator 4 is fed to an arithmetic logic unit 5. The arithmetic logic unit 5 counts the period (T sec.) of the pulse signal fed thereto and determines the heart-beat rate from the number of pulses per minute. The relationship between the period (T sec.) and the pulse signal and the number P of heart-beats per minute, i.e. heart-beat rate, is given by:

$$P = 60/T$$

The output signal from the arithmetic logic unit 5 is fed to a display circuit 6 and drives a device (not shown), for example, a liquid crystal display device, to display the heart-beat.

To conserve power the heart-beat rate indicator may be provided with a switching circuit for controlling the energy supply from a power source (not shown), the switching circuit being in an OFF state when the heart-beat rate is not being measured.

However, an input transistor of the operational amplifier of the amplifier circuit 2 may be destroyed or damaged if the detection electrode 1 receives an electrostatic charge. Such electrostatic charge is readily produced, for example, by putting on or taking off clothing in daily life. Electrostatic charge built up on the body surface is discharged when the detection electrode 1 is touched irrespective of whether the heart-beat rate indicator is in use or not.

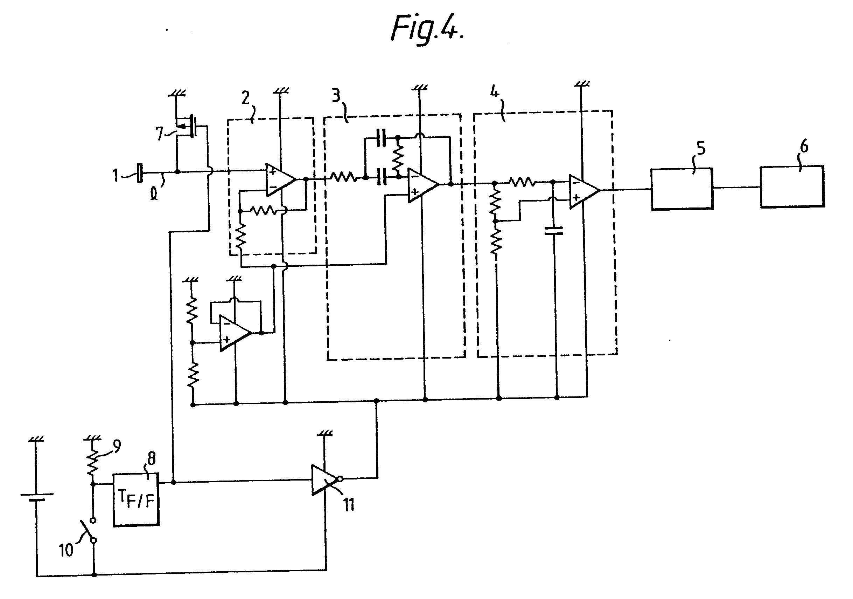

Figure 3 illustrates one embodiment of a heart-beat rate indicator according to the present invention. Further description of the detection electrode 1, the amplifier circuit 2, the band-pass filter 3, the voltage comparator 4, the arithmetic logic unit 5, the display circuit 6 is omitted.

A positive input of the amplifier circuit 2 is connected to the detection electrode as well as to ground potential through a switching element 7. The switching element 7 in this embodiment consists of a P-channel MOSFET. The source electrode of the switching element is connected to ground potential, the drain electrode is connected to the positive input of the amplifier circuit 2, and the gate electrode is connected to an output of a T-type flip-flop circuit 8 which acts as a memory device. The input of the flip-flop circuit 8 is connected to one side of a resistor 9. The other side of the resistor 9 is connected to ground potential. One terminal of a switch 10 is connected to the input of the flip-flop cicuit 8 and the terminal is connected to a negative side of the power source. In this embodiment, the ground potential is the positive side of the power source.

An output signal from the flip-flop circuit 8 is level O when the heart-beat rate indicator is not in use and the switching element 7 is conductive. The resistance value of the switching element 7 in the conductive state is designed to be small. The detection electrode 1 thus is connected to groud potential and eletrostatic charge discharged to the detection electrode 1 flows to ground through the switching element. Consequently, the amplifier circuit 2 is not damaged or destroyed by the electrostatic charge.

When the heart-beat rate indicator is to be used, the switch 10 is pressed so that it closes and opens again resulting in a pulse being applied to the input of the flip-flop circuit 8 whose output signal becomes level 1. This causes the switching element 7 to become non-conductive and the detection electrode 1 disconnected from ground potential. The heart-beat rate indicator thus operates as that shown in Figure 2. The user presses the switch 10 again on finishing measurement of heart-beat rate, producing another pulse which is applied to the input of the flip-flop circuit 8 so that its output signal becomes level O and the switching element becomes conductive. Thus the heart-beat rate indicator is again protected from damage by electrostatic charge.

Figure 4 illustrates another embodiment of a heart-beat rate indicator according to the present invention. Like parts in Figures 3 and 4 have been designated by the same reference numerals and will not be described further in detail. A buffer 11 is connected to the output of the flip-flop circuit 8 and an output of the buffer is connected to a negative side of the amplifier circuit 2, the band-pass filter 3 and the voltage comparator 4. The buffer 11 has a logic inverting function. When the heart-beat rate indicator is not in use and the output signal from the flip-flop circuit 8 is level O, the output signal from the buffer 11 is level 1 and no current flows in the amplifier circuit 2, the band-pass filter 3 and the voltage comparator 4. When the heart-beat rate indicator is in use and the output signal from the flip-flop circuit 8 is level 1, the output signal from the buffer 11 is level O and current can flow in the amplifier circuit 2, the band-pass filter 3 and the voltage comparator 4. The circuitry of the heart-beat rate indicator is thus protected from damage from electrostatic charge and power consumption is reduced by the provision of the switch 10.

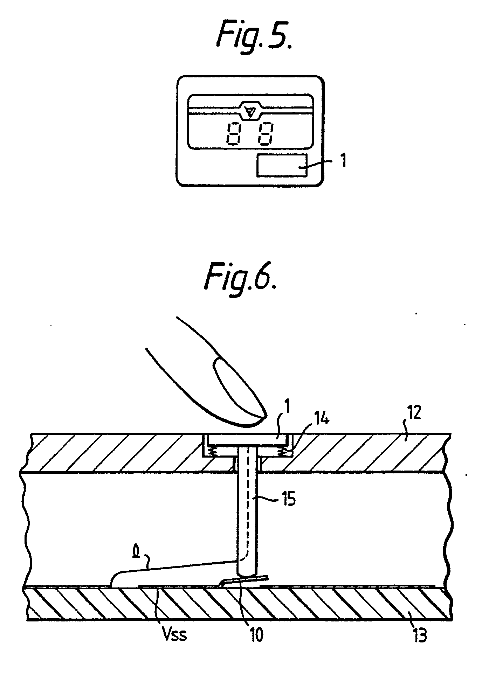

Figure 5 is a plan view of a display device of a heart-beat rate indicator according to the present invention.

Figure 6 is a sectional view of the detection electrode 1 and the switch 10. The detection electrode 1 is mounted *via* a spring 14 on a case 12 of the heart-beat rate indicator and is connected by lead I to an input of the amplifier circuit 2. A portion of the lead I passes through an electrically insulating member 15 which operates the switch 10 whose contacts are mounted on a printed circuit board 13.

The heart-beat rate indicators according to the present invention and described above are highly reliable and have the advantage that the detection electrode can be connected to ground potential so that the circuitry can be protected from damage by electrostatic charge.

It will be appreciated that electrostatic charge transferred to the detection electrode is eliminated before the switch 10 is actuated.

**Claims**

1. A heart-beat rate indicator comprising: a detection electrode (1) for receiving an electrocardiac potential signal; and circuit means (2, 3, 4, 5, 6) for receiving the electrocardiac potential signal for determining therefrom a heart-beat rate; characterised by a switching element (7) one side of which is connected to the detection electrode and an input of the circuit means and the other side of which is connectable to ground potential, switching means (8, 9, 10) for controlling the conductive state of the switching element (7), the switching means including a mechanical switch (10) operated by pressure applied to the detection electrode (1) and arranged to ensure electrostatic charge transferred to the detection electrode is eliminated before the mechanical switch (10) is actuated.

2. A heart-beat rate indicator as claimed in claim 1 characterised in that the switching element (7) is a P-channel MOSFET.

3. A heart-beat rate indicator as claimed in claim 1 or 2 characterised in that the switching means includes memory means (8) connected to control the conductive state of the switching element (7).

4. A heart-beat rate indicator as claimed in claim 3 characterised in that the memory means (8) is connected to control the conductive state of the circuit means (2, 3, 4, 5, 6).

5. A heart-beat rate indicator as claimed in claim 3 or 4 characterised in that the memory means (8) is a flip-flop circuit.

## Patentansprüche

1. Herzfrequenzindikator mit einer Detektorelektrode (1) zur Aufnahme eines elektrokardiographischen Spannungssignals und mit einer Schaltungsanordnung (2, 3, 4, 5, 6) zur Aufnahme des elektrokardiographischen Spannungssignals zwecks Ermittlung der Herzfrequenz daraus, gekennzeichnet durch ein Schaltelement (7), das mit einer Seite mit der Detektorelektrode und einem Eingang der Schaltungsanordnung verbunden und mit der anderen Seite mit Erdpotential verbindbar ist und durch eine Schaltanordnung (8, 9, 10) zur Steuerung des leitenden Zustandes des Schaltelementes (7), welche einen durch auf die Detektorelektrode (1) wirkenden Druck betätigen mechanischen Schalter (10) enthält und so ausgebildet ist, daß auf die Detektorelektrode übertragene elektrostatische Ladung vor der Betätigung des mechanischen Schalters (10) sicher eliminiert wird.

2. Herzfrequenzindikator nach Anspruch 1, dadurch gekennzeichnet, daß das Schaltelement (7) ein P-Kanal-MOS-Feldeffekttransistor ist.

3. Herzfrequenzindikator nach Anspruch 1 oder 2, dadurch gekennzeichnet, daß die Schaltanordnung einen zur Steuerung des leitenden Zustandes des Schaltelements (7) geschalteten Speicher (8) enthält.

4. Herzfrequenzindikator nach Anspruch 3, dadurch gekennzeichnet, daß der Speicher (8) zur Steuerung des leitenden Zustandes der Schaltungsanordnung (2, 3, 4, 5, 6) geschaltet ist.

5. Herzfrequenzindikator nach Anspruch 3 oder 4, dadurch gekennzeichnet, daß der Speicher (8) eine Flipp-Flopp-Schaltung ist.

## Revendications

1. Indicateur de rythme cardiaque comprenant: une électrode de détection (1) servant à recevoir un signal de potentiel électrocardiaque; et un circuit (2, 3, 4, 5, 6) servant à recevoir le signal de potentiel électrocardiaque afin de déterminer à partir de celui-ci le rythme cardiaque; caractérisé par un élément de commutation (7) dont un premier cté est connecté à l'électrode de détection et à une entrée du circuit et dont l'autre cté peut être connecté au potentiel de la terre, un moyen de commutation (8, 9, 10) servant à commander l'état conducteur de l'élément de commutation (7), le moyen de commutation comportant un commutateur mécanique (10) actionné par pression s'appliquant à l'électrode de détection (1) et conçu pour assurer que la charge électrostatique transférée à l'électrode de détection a été éliminée avant l'actionnement du commutateur mécanique (10).

2. Indicateur de rythme cardiaque selon la revendication 1, caractérisé en ce que l'élement de commutation (7) est un MOSFET à canal P.

3. Indicateur de rythme cardiaque selon la revendication 1 ou 2, caractérisé en ce que le moyen de commutation comporte un moyen de mémoire (8) connecté afin de commander l'état conducteur de l'élément de commutation (7).

4. Indicateur de rythme cardiaque selon la revendication 3, caractérisé en ce que le moyen de mémoire (8) est connecté afin de commander l'état conducteur du circuit (2, 3, 4, 5, 6).

5. Indicateur de rythme cardiaque selon la revendication 3 ou 4, caractérisé en ce que le moyen de mémoire (8) est un circuit basculeur.

Fig.1.

Fig.2.

Fig.3.

Fig.4.

0 085 577

## Fig.5.

1

## Fig.6.

1
14
12
15
0
Vss
10
13

3